# EUROPÄISCHE PATENTSCHRIFT

(11) **EP 0 641 857 B1**
(45) Veröffentlichungstag und Bekanntmachung des Hinweises auf die Patenterteilung: **31.10.2001**
(21) Anmeldenummer: 93111153.8
(22) Anmeldetag: 13.07.1993
(51) Int. Cl.: C12N 1/20, A23B 4/22, A23L 1/314

(54) **Zum Reifen von Rohwurst geeignete Mikroorganismen vom Stamm Lactobacillus sake**
Lactobacillus sake strain useful for curing raw sausage
Souche de lactobacillus sake utile pour salage de saucisse crue

(43) Veröffentlichungstag der Anmeldung: 08.03.1995
(73) Patentinhaber: Karl Müller & Co. KG, 70469 Stuttgart (DE)
(72) Erfinder: Hammes, Walter Prof.Dr., 70794 Filderstadt (DE)
(74) Vertreter: Thiel, Christian, Dr. Dipl.-Chem.

(56) Entgegenhaltungen:
- EP-A- 0 321 692
- DE-A- 4 035 836
- DE-C- 4 201 050
- DATABASE WPI Week 9240, Derwent Publications Ltd., London, GB; AN 92-327608 & JP-A-4 234 963 (SANEI TOKA KK) 24. August 1992

## Beschreibung

Die Erfindung betrifft einen neuen Mikroorganismus der Art Lactobacillus sake, der insbesondere zur Reifung von Fleischwaren geeignet ist. Der neue Mikroorganismus wurde bei der deutschen Sammlung von Mikroorganismen und Zellkulturen nach den Bestimmungen des Budapester Vertrags hinterlegt und wird dort unter der Eingangsnummer DSM 6747 geführt.

Es ist seit geraumer Zeit bekannt, daß zur Fermentierung von rohen Pökelfleischwaren (Absäuerung) Milchsäurebildner als Starterkulturen eingesetzt werden. Diese konventionellen Säurebildner entstammen in der Regel fremden Biotopen und sind im allgemeinen auf ihre Leistungsfähigkeit und ihre Darstellungsmöglichkeit hin selektiert.

DE-C1-37 39 989 beschreibt den Mikroorganismus vom Stamm Lactobacillus curvatus DSM 4265, die zur Herstellung von Pökelfleischwaren geeignet sind. Dieser Mikroorganismus wird in einer Menge von 5 X 10⁹ Keimen pro kg Wurstmasse in gefriergetrocknetem Zustand zugesetzt. Die Reifung unter kontrollierten Klimabedingungen dauert etwa 10 Tage und führt zu einer Absenkung des pH-Werts, zur Ausbildung eines typischen Aromas sowie zu einer Stabilisierung der Rohwurst durch Behinderung der spontanen Säurebildnerflora.

Insgesamt hat sich jedoch gezeigt, daß der Reifungsprozeß mit herkömmlichen Lactobacillen trotz relativ hoher Reifungstemperaturen von bis zu 25°C relativ langwierig ist.

Dieser lange Reifungsprozeß begünstigt die Ausbildung einer konkurrierenden Spontanflora, was nicht nur unter hygienischen Gesichtspunkten unerwünscht ist, sondern auch unter dem Gesichtspunkt der Kontrollierbarkeit des Reifungsprozesses.

Weiterhin werden zur Stabilisierung von Fleischwaren Mikroorganismen vom Stamme Pediococcus pentosaceus eingesetzt, die ebenfalls zur Ausbildung von Milchsäure und zur Absenkung des pH-Werts führen. Die Verwendung dieser Mikroorganismen ist beispielsweise in der bereits oben erwähnten DE-C1-37 39 989, Beispiel 3, beschrieben. Dieser Mikroorganismus führt jedoch zu einer nicht für alle Produkte als optimal zu bezeichnenden unzureichenden Rötung des Produkts, sofern nicht geeignete Maßnahmen getroffen werden. Als solche Maßnahme gilt die Zugabe von Hefe, beispielsweise der Art Debariomyces hansenii, die innerhalb der Fermentierungszeit von 24 bis 36 Stunden bis hin zu einem End-pH-Wert der fertigen Wurst von 5,4 bis 5,2 zu einer ausreichenden Umrötung der Wurstmasse führt. Der Zusatz dieses Hefestamms führt dabei aber zu einem typischen Eigengeschmack der Wurst. Auch die Umrötung ist hinsichtlich ihrer Beständigkeit nicht immer optimal.

Die bekannten Mikroorganismen, die zur Reifung von Rohwurst eingesetzt werden, benötigen für eine maximale Aktivität eine recht hohe Temperatur und einen nicht zu stark abgesenkten pH-Wert. Weiterhin wird ihre Aktivität durch eine zu hohe Kochsalzkonzentration eingeschränkt.

Ziel der Erfindung ist die Bereitstellung eines Mikroorganismus, der bei ausreichender Kochsalztoleranz und Beständigkeit gegen niedrige pH-Werte auch bei unterhalb der bisherigen Reifungstemperaturen von Wurstwaren liegenden Temperaturen eine ausreichende Reifungsgeschwindigkeit gewährleistet. Darüber hinaus ist eine durchgehende Umrötung der behandelten Rohwurst wünschenswert wie auch eine stabile Pökelfarbe über die Haltbarkeitsdauer der Wurstware.

Dieses Ziel wird mit einem Mikroorganismus der eingangs genannten Art erreicht, der insbesondere für die Reifung von schnittfester Rohwurst geeignet ist, aber auch für die Erzeugung streichfähiger Rohwurst.

Die Erfindung betrifft ferner ein Mittel zum Reifen von Rohwurst, das wenigstens einen Mikroorganismus vom Stamm Lactobacillus sake DSM 6747 enthält. Zweckmäßigerweise können diesem Mittel weitere Mikroorganismen zugesetzt sein, insbesondere ein Stamm von Staphylococcus carnosus, wie er üblicherweise bei der Wurstherstellung verwandt wird. Mit einem solchen weiteren Stamm wird eine optimale Umrötung des Produkts erreicht.

Zweckmäßigerweise enthält das Mittel die Stämme Lactobacillus sake DSM 6747 und Staphylococcus carnosus in einem Verhältnis, bezogen auf die Keimzahl von 20:80 bis 80:20, vorzugsweise etwa 50:50. Es ist ferner zweckmäßig, daß das Reifungsmittel die Mikroorganismen in gefriergetrocknetem Zustand, gegebenenfalls neben üblichen weiteren Bestandteilen, enthält. Übliche weitere Bestandteile sind beispielsweise Nährstoffe und -salze.

Das erfindungsgemäße Mittel wird in einer Menge von 20 bis 100 g pro 100 kg Wurstmasse eingesetzt, vorzugsweise etwa 50 g pro 100 kg. Dies entspricht einer Menge von 10⁸ bis 10¹¹ Keimen/kg Wurstmasse, insbesondere etwa 10⁹ bis 10¹⁰ Keimen. Besonders bevorzugt ist eine Menge von etwa 5 X 10⁹ Keimen des Stammes Lactobacillus sake DSM 6747 und etwa 5 X 10⁹ Keimen der Spezies Staphylococcus carnosus pro kg Wurstmasse.

Der neuentwickelte Stamm von Lactobacillus sake zeigt optimale Fermentationseigenschaften, auch unter bei der Wurstherstellung extremen Bedingungen. Eine gute Katalaseaktivität führt zu einer Farb- und Aromastabilisierung beim Produkt. Der Stamm hat ferner den Vorteil, daß er über eine Plasmiduntersuchung schnell und leicht untersucht bzw. nachgewiesen werden kann.

Das erfindungsgemäße Reifungsmittel führt ferner bei einer beschleunigten Säuerung der damit behandelten Rohwurst zu einer kontrollierten und standardisierten Fermentation. Durch eine Behinderung der spontanen Mikroorganismenflora wird das hygienische Risiko vermindert und die Ausbildung eines für den erfindungsgemäßen Mikroorganismus charakteristischen Fermentationsaromas erzielt.
Die bei der Fermentation ausgebildete typische Pökelfarbe erweist sich über die Haltbarkeitsdauer der Wurst als überdurchschnittlich stabil.

Von den beigefügten Abbildungen zeigt
- Fig. 1 A bis E: das Wachstumsverhalten verschiedener Mikroorganismen in Abhängigkeit von der Temperatur, wobei Fig. 1 C die Daten für Lactobacillus sake DSM 6747 angibt;
- Fig. 2 A bis H: den Einfluß des pH-Wertes auf die Wachstumsgeschwindigkeit verschiedener Mikroorganismen, wobei die Werte für Lactobacillus sake DSM 6747 in Fig. 2 C dargestellt sind;
- Fig. 3 A bis H: den Einfluß der NaCl-Konzentration auf die Wachstumsgeschwindigkeit verschiedener Mikroorganismen, darunter Lactobacillus sake DSM 6747 in Fig. 3 G sowie
- Fig. 4: die Säuerungsgeschwindigkeit verschiedener Mikroorganismen in Abhängigkeit von der Zeit.

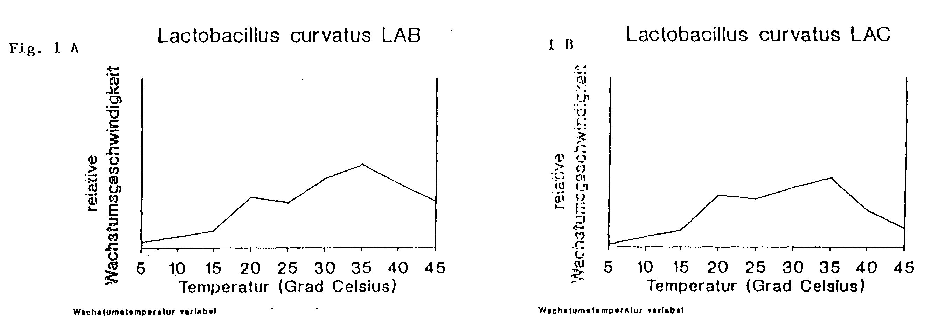

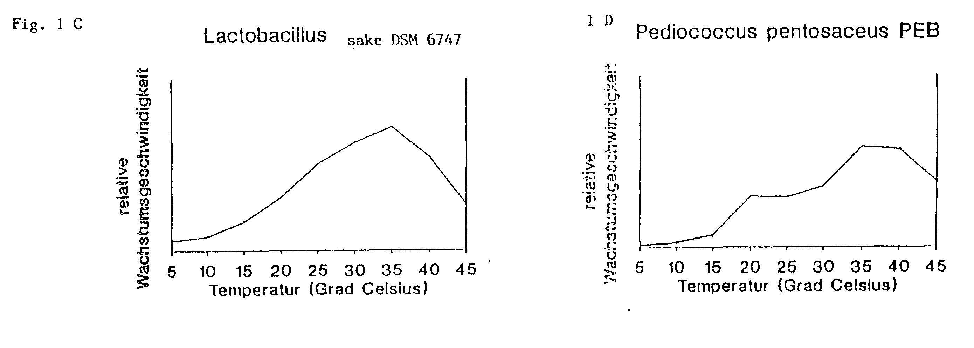

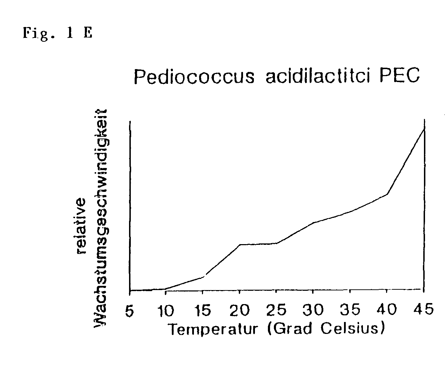

Der in Fig. 1 dargestellte Einfluß der Temperatur auf die Wachstums- und Säuerungsgeschwindigkeit verschiedener Mikroorganismen gegen einen externen Standard zeigt für den erfindungsgemäßen Lactobacillus sake DSM 6747 einen kontinuierlichen Anstieg von 5° bis 35°C. Gegenüber anderen zur Vermentierung von Rohwurst verwandten Mikroorganismen hat der erfindungsgemäße Mikroorganismus die bei der üblichen Fermentierungstemperatur von 25°C absolut höchste Wachstumsgeschwindigkeit. Die kontinuierliche Abnahme der Werte bis zu einer Temperatur von 5°C erlaubt eine einfache Steuerung der Fermentierung bei der jeweilig erwünschten Temperatur, wobei die vergleichsweise relativ hohen Geschwindigkeiten jeweils eine relativ kurze Reifungszeit ermöglichen. Die Wachstumsgeschwindigkeit ist selbst bei 5°C der aller anderen untersuchten Mikroorganismen überlegen.

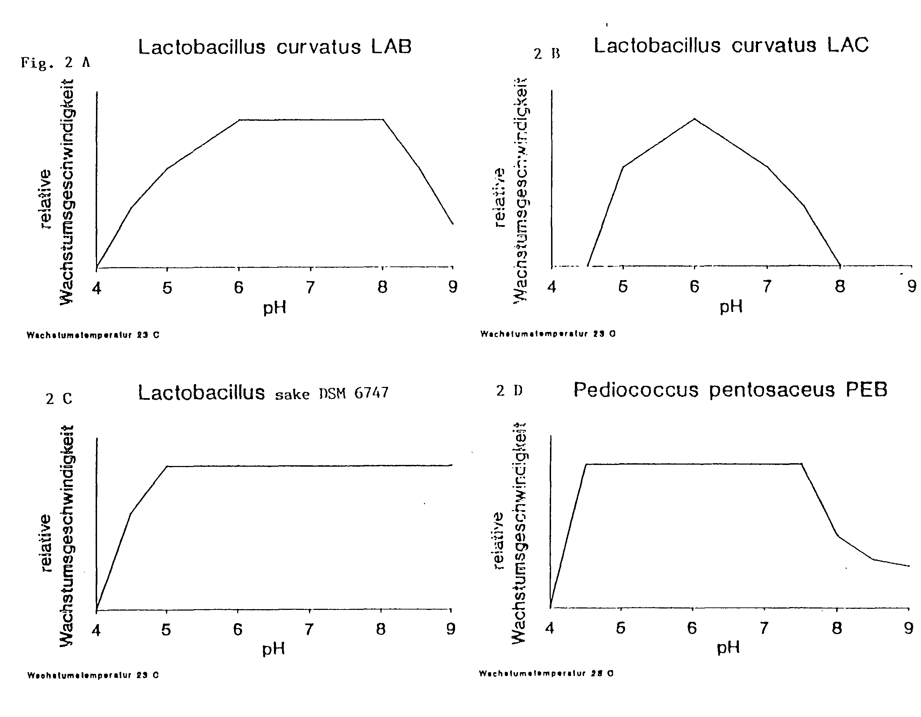

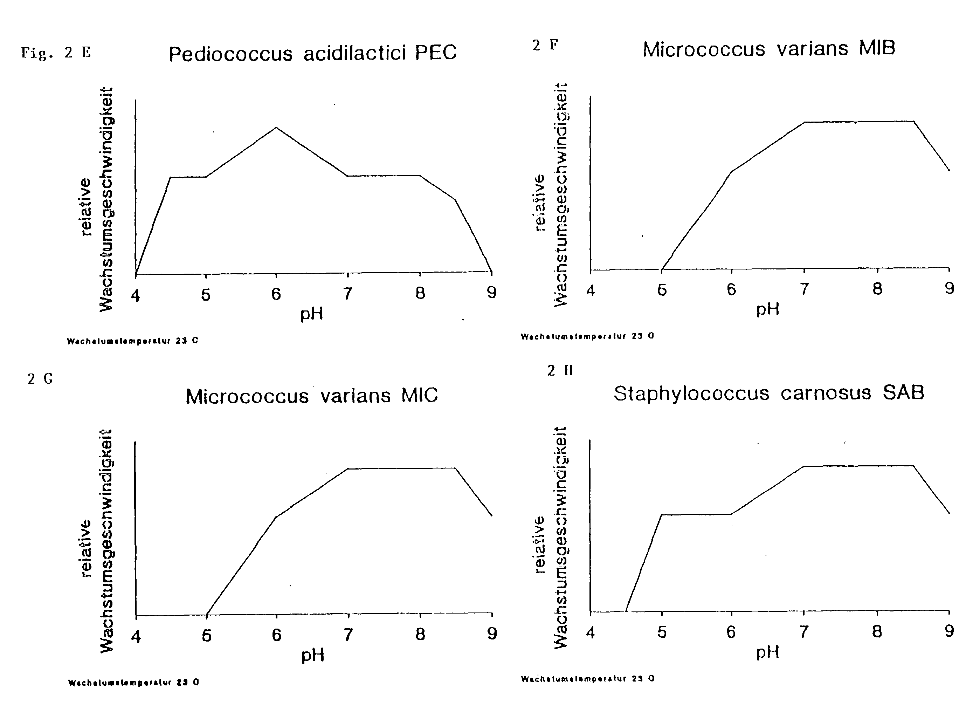

Wie sich aus Fig. 2 ergibt, ist die relative Wachstumsgeschwindigkeit des erfindungsgemäßen Mikroorganismus bis hinunter zu einem pH-Wert von 5 im wesentlichen pH-unabhängig. Lediglich Pediococcus pentosaceus PEB zeigt eine vergleichbar günstige Wachstumsgeschwindigkeit bei niedrigen pH-Werten.

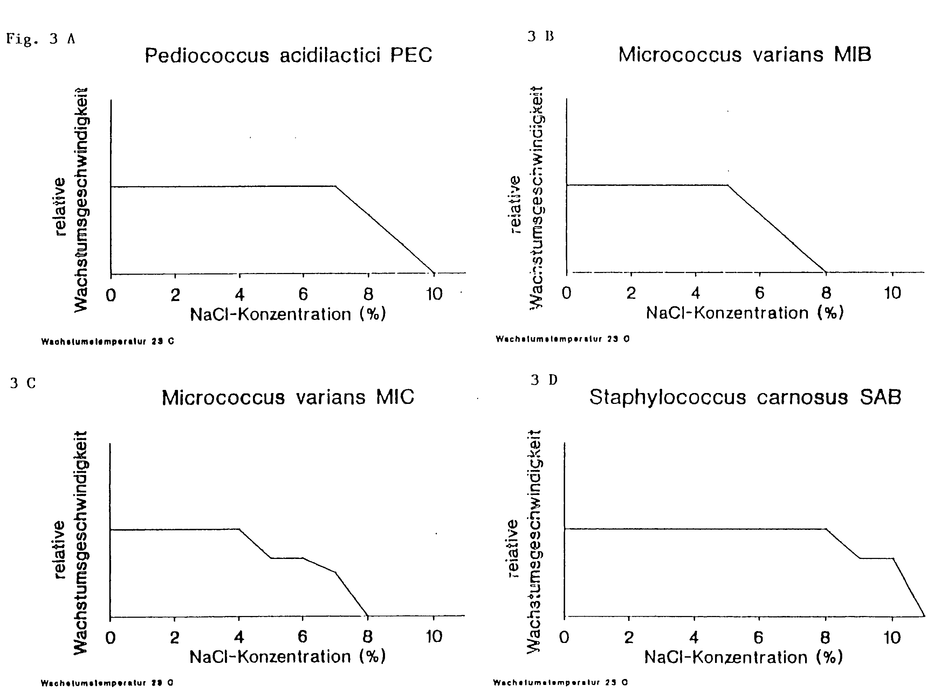

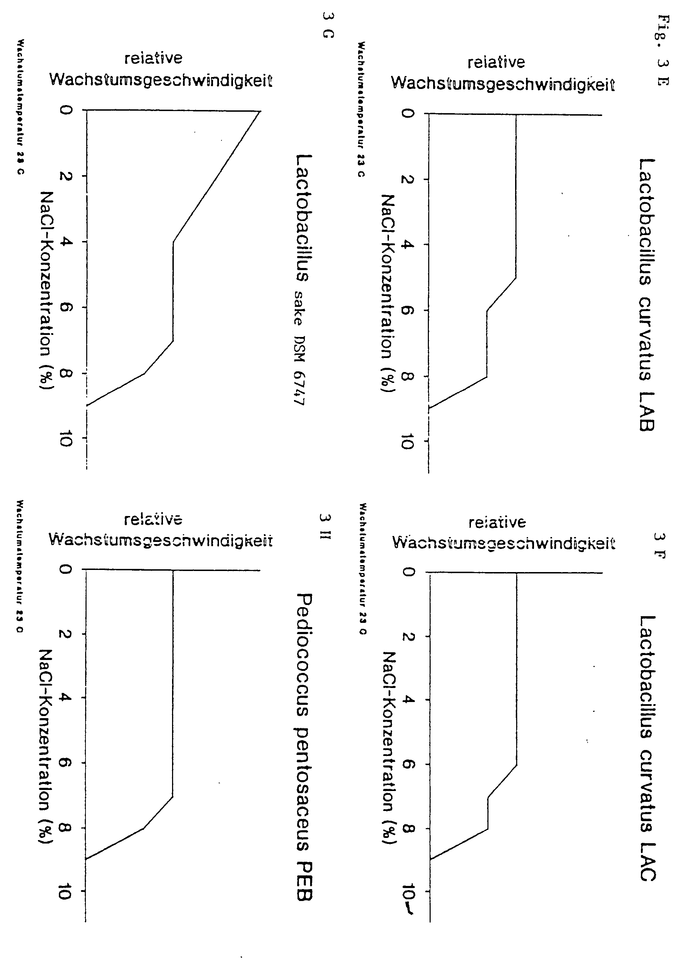

Fig. 3 zeigt die relative Wachstumsgeschwindigkeit des erfindungsgemäßen Mikroorganismus in Abhängigkeit von der NaCl-Konzentration im Vergleich zu einer Reihe herkömmlicher, bei der Fermentierung von Rohwurst verwandter Mikroorganismen. Die überlegene Wachstumsgeschwindigkeit des erfindungsgemäßen Stamms von Lactobacillus sake bei niedrigen NaCl-Konzentrationen bis zu 4 % ist deutlich zu erkennen. Die Kochsalztoleranz ist eine Eigenschaft, die insbesondere für die Herstellung stärker gesalzener Wurstwaren von Bedeutung ist, denen Kochsalz in Mengen von bis zu 6 Gew.-% zugesetzt werden darf.

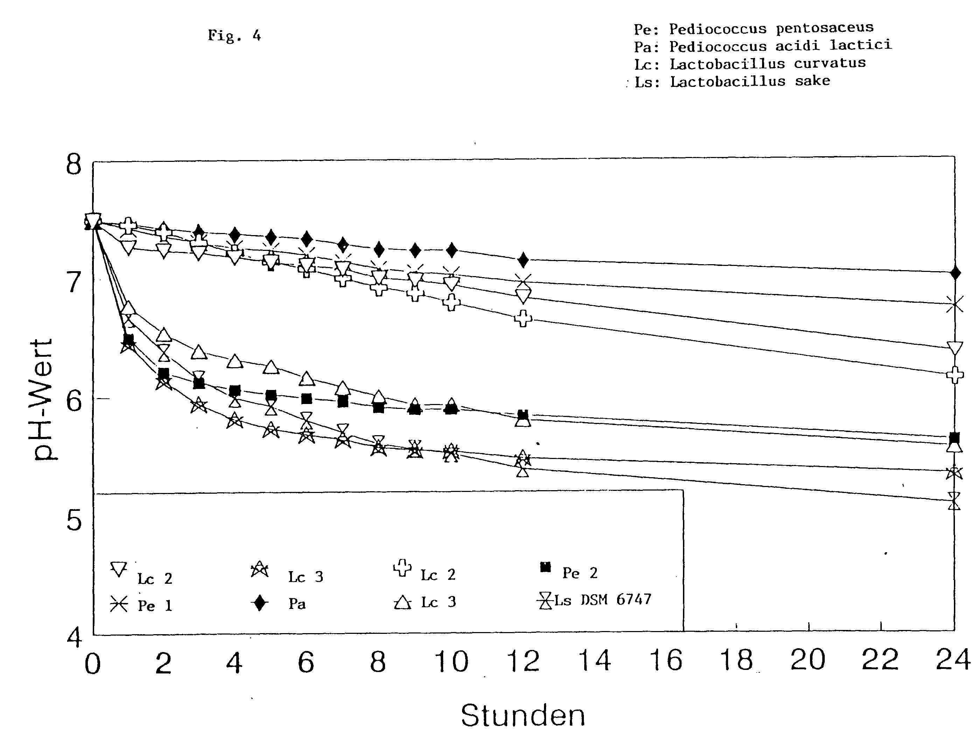

Fig. 4 zeigt das Reaktivierungsverhalten von Starterkulturmischungen einer Reihe herkömmlicher Mikroorganismen im Vergleich zu dem erfindungsgemäßen Lactobacillus sake DSM 6747. Dazu wird die Säuerungsgeschwindigkeit nach Einrühren von 50 g Starterkulturmischung und 500 ml Wasser mit einem herkömmlichen Nährmedium bei einer Inkubationstemperatur von 4°C in Abhängigkeit von der Zeit gemessen. Lediglich ein Stamm von Lactobacillus curvatus LC3 zeigt ein ähnlich gutes Reaktivierungsverhalten mit einem starken Abfall des pH-Werts in den ersten 10 Stunden; insgesamt werden mit dem erfindungsgemäßen Mikroorganismus über die Meßdauer von 24 Stunden die niedrigsten pH-Werte erhalten, was das günstige Säuerungsverhalten dieses Mikroorganismus unterstreicht.

Die Erfindung wird durch das folgende Ausführungsbeispiel näher erläutert.

### Beispiel

### Lactobacillus sake DSM 6747 bei der Herstellung von Salami (schnittfeste Rohwurst)

### Materialzusammenstellung

30 kg Rindfleisch, grob entsehnt, sichtbarer Fettanteil 5 %,
30 kg Schweinefleisch, sehnenfrei, sichtbarer Fettanteil 5 %,
20 kg Schweinebauch, sichtbarer Fettanteil 60 %,
20 kg Rückenspeck, ohne Schwarte

### Zusätze je 1 kg Material

28,0 g Kochsalz
0,3 g Kaliumnitrat
4,0 g Glukose
2,0 g Pfeffer, weiß, gemahlen
5 X 10⁹ Lactobacillus sake DSM 6747, gefriergetrocknet
5 X 10⁹ Staphylococcus carnosus, gefriergetrocknet (zur Optimierung der Umrötung, handelsübliche Qualität)

Zur Herstellung der Rohwurstmasse wurden die Materialien jeweils in Stücke geschnitten, das Rindfleisch, der Schweinebauch und der Speck hartgefroren und das Schweinefleisch gut durchgekühlt und unmittelbar vor der Verarbeitung in üblicher Weise gewolft.

Die gefriergetrockneten Starterkulturen Lactobacillus sake DSM 6747 und Staphylococcus carnosus wurden in 500 ml Wasser mit einer üblichen Reaktivierungsmischung eingerührt.

Das Rindfleisch wurde bei Zugabe der Starterkulturen im Kutter so lange vorzerkleinert, bis die Masse etwas bindet. Danach wurden das Kaliumnitrat, die Glukose sowie der Pfeffer hinzugefügt und die Masse noch eine kurze Zeit im Kutter weiter laufengelassen, um eine ausreichende Vermischung zu erzielen. Danach wurden der Speck und der Schweinebauch zugesetzt. Es wurde so lange weitergekuttert, bis der Speck eine Körnung von 6-8 mm aufwies. Anschließend wurde das vorgewolfte Schweinefleisch mit dem Kochsalz eingekuttert und die Gesamtmasse so lange weitergekuttert, bis das Fett die übliche Körnung von etwa 2 mm und die Wurstmasse Bindung aufwies.

Die fertige Wurstmasse mit einer Temperatur von -2°C wurde in wasserdurchlässige Hautfaserdärme vom Kaliber 70 mm eingefüllt. Anschließend wurden die gefüllten Därme zur Reifung in die Klimakammer verbracht.

Zunächst wurden die Würste bei 70 % relativer Luftfeuchtigkeit und etwa 18°C Temperatur 6 Stunden vorkonditioniert, um das äußere Schwitzwasser wegzutrocknen. Danach wurde zur weiteren Konditionierung die relative Luftfeuchtigkeit 18 Stunden auf 94 % erhöht. Die eigentliche Reifung erfolgte bei einer Anfangstemperatur von 24°C über einen Zeitraum von 36 bis 48 Stunden, wobei die Temperatur und Luftfeuchtigkeit langsam auf übliche Bedingungen zurückgenommen und die Würste fertiggeraucht wurden.

Nach der Trocknung und der üblichen Nachreifebedingungen wurde ein pH-Wert von 5,2 bis 5,0 festgestellt. Die Würste wiesen eine hervorragende Umrötung sowie eine sehr haltbare und ansprechende Pökelfarbe im Anschnitt auf.

## Patentansprüche

1. Lactobacillus sake DSM 6747, insbesondere zum Reifen von Fleischwaren.

2. Mittel zum Reifen von Rohwurst, **dadurch gekennzeichnet, daß** es Mikroorganismen vom Stamm Lactobacillus sake DSM 6747 enthält.

3. Mittel nach Anspruch 2, **dadurch gekennzeichnet, daß** es den Mikroorganismus Lactobacillus sake DSM 6747 zusammen mit wenigstens einem weiteren Mikroorganismus enthält.

4. Mittel nach Anspruch 3, **dadurch gekennzeichnet, daß** es als weiteren Mikroorganismus einen Stamm von Staphylococcus carnosus enthält.

5. Mittel nach Anspruch 4, **dadurch gekennzeichnet, daß** es Mikroorganismen der Stämme Lactobacillus sake DSM 6747 und Staphylococcus carnosus im Verhältnis 20:80 bis 80:20 enthält.

6. Mittel nach Anspruch 5, **dadurch gekennzeichnet, daß** das Verhältnis etwa 50:50 ist.

7. Mittel nach einem der Ansprüche 2 bis 6, **dadurch gekennzeichnet, daß** es die Mikroorganismen in gefriergetrocknetem Zustand enthält.

8. Verwendung von Lactobacillus sake DSM 6747 zum Reifen von Rohwurst.

9. Verwendung nach Anspruch 8 in einer Menge von 10⁸ bis 10¹¹ Keimen/kg Rohwurstmasse.

10. Verwendung nach Anspruch 9 in einer Menge von 10⁹ bis 10¹⁰ Keimen/kg Rohwurstmasse.

11. Verwendung nach einem der Ansprüche 8 bis 10 in einer Menge von 5 x 10⁹ Keimen des Stammes Lactobacillus sake DSM 6747 zusammen mit etwa 5 x 10⁹ Keimen eines Stammes von Staphylococcus carnosus.

12. Verwendung nach einem der Ansprüche 8 bis 11 zusammen mit bei der Reifung von Wurstwaren üblichen Würz-, Aroma- und Zusatzstoffen.

## Claims

1. Lactobacillus sake DSM 6747, particularly for maturing meat products.

2. Compound for maturing raw sausage, **characterised in that** it contains microorganisms of the strain Lactobacillus sake DSM 6747.

3. Compound as claimed in claim 2, **characterised in that** it contains the microorganism Lactobacillus sake DSM 6747 together with at least one further microorganism.

4. Compound as claimed in claim 3, **characterised in that** it contains a strain of Staphylococcus carnosus as the further microorganism.

5. Compound as claimed in claim 4, **characterised in that** it contains microorganisms of the strains Lactobacillus sake DSM 6747 and Staphylococcus carnosus in the ratio 20:80 to 80:20.

6. Compound as claimed in claim 5, **characterised in that** the ratio is about 50:50.

7. Compound as claimed in one of claims 2 to 6, **characterised in that** it contains the microorganisms in freeze dried state.

8. Use of Lactobacillus sake DSM 6747 for maturing raw sausage.

9. Use as claimed in claim 8 in an amount of 10⁸ to 10¹¹ bacteria/kg raw sausage mass.

10. Use as claimed in claim 9 in an amount of 10⁹ to 10¹⁰ bacteria/kg raw sausage mass.

11. Use as claimed in one of claims 8 to 10 in an amount of 5 x 10⁹ bacteria of the strain Lactobacillus sake DSM 6747 together with about 5 x 10⁹ bacteria of a strain of Staphylococcus carnosus.

12. Use as claimed in one of claims 8 to 11 together with seasoning, aroma and additive materials conventional in the maturation of sausage products.

## Revendications

1. Lactobacillus sake DSM 6747, en particulier pour le mûrissement de produits à base de viande.

2. Produit pour le mûrissement de saucisse crue, **caractérisé en ce qu'**il contient des micro-organismes de souche Lactobacillus sake DSM 6747.

3. Produit selon la revendication 2, **caractérisé en ce qu'**il contient le micro-organisme Lactobacillus sake DSM 6747 conjointement avec au moins un autre micro-organisme.

4. Produit selon la revendication 3, **caractérisé en ce qu'**il contient comme autre organisme une souche de Staphylococcus carnosus.

5. Produit selon la revendication 4, **caractérisé en ce qu'**il contient des micro-organismes des souches Lactobacillus sake DSM 6747 et Staphylococcus carnosus dans le rapport 20:80 à 80:20.

6. Produit selon la revendication 5, **caractérisé en ce que** le rapport est d'environ 50:50.

7. Produit selon l'une des revendications 2 à 6, **caractérisé en ce qu'**il contient les micro-organismes à l'état lyophilisé.

8. Utilisation du Lactobacillus sake DSM 6747 pour le mûrissement de saucisse crue.

9. Utilisation selon la revendication 8 en une quantité de 10⁸ à 10¹¹ germes par kg de masse de saucisse crue.

10. Utilisation selon la revendication 9 dans une quantité de 10⁹ à 10¹⁰ germes par kg de masse de saucisse crue.

11. Utilisation selon l'une des revendications 8 à 10 dans une quantité de 5 x 10⁹ germes de la souche Lactobacillus sake DSM 6747 conjointement avec environ 5 x 10⁹ germes d'une souche de Staphylococcus carnosus.

12. Utilisation selon l'une des revendications 8 à 11 conjointement avec des condiments, des arômes et des additifs classiques dans le mûrissement des produits à base de saucisse.
